# EUROPEAN PATENT SPECIFICATION

(11) **EP 1 014 918 B1**
(45) Date of publication and mention of the grant of the patent: **05.04.2006**
(21) Application number: 98948186.6
(22) Date of filing: 15.09.1998
(51) Int. Cl.: A61K 8/34, A61Q 5/06

(54) **IMPROVED HAIR SPRAY AND CONSUMER SPRAYS WITH REDUCED VOLATILE ORGANIC COMPOUNDS**
HAARSPRAY UND VEBRAUCHSSPRAY MIT VERMINDERTEM GEHALT AN FLÜCHTIGEN ORGANISCHEN VERBINDUNGEN
COMPOSITION PULVERISABLE FIXATRICE DE CHEVEUX ET COMPOSITIONS PULVERISABLES DE CONSOMMATION PERFECTIONNEES A COMPOSES ORGANIQUES VOLATILS REDUITS

(30) Priority: 17.09.1997 US 59764 P; 20.01.1998 US 71909 P
(43) Date of publication of application: 05.07.2000
(73) Proprietor: EASTMAN CHEMICAL COMPANY, Kingsport, TN 37660 (US)
(72) Inventor: DOBBS, Suzanne, W., Kingsport, TN 37660-3417 (US); OLDFIELD, Terry, A., Kingsport, TN 37660-4761 (US); COOK, Phillip, M., Kingsport, TN 37664-4780 (US); BRYAN, William, R., Kingsport, TN 37664-2054 (US)
(74) Representative: Wibbelmann, Jobst
(86) International application number: PCT/US1998/019073
(87) International publication number: WO 1999/013836

(56) References cited:
- EP-A- 0 858 795
- US-A- 4 173 627
- US-A- 4 243 548
- US-A- 5 094 838
- PATENT ABSTRACTS OF JAPAN vol. 96, no. 11, 29 November 1996 & JP 08 187277 A (POLA), 23 July 1996

## Description

This invention relates generally to consumer sprays that contain ethanol as a solvent for active ingredients in the spray, and to the replacement of ethanol by alkyl acetates, especially methyl acetate and t-butyl acetate. The invention also has application to consumer sprays that contain isopropanol, and the replacement of isopropanol by these alkyl acetates.

### BACKGROUND OF THE INVENTION

Hair sprays were introduced to the public in the late 1940's and were an immediate success. The first sprays were pump type systems, but public demand for a better product caused the development of the aerosol spray. The aerosol spray was based on Goodhue and Sullivan's patent of low-pressure propellants (fluorocarbon 12) and Abplanalp's patented valve design. The first aerosol hair spray was introduced to the public in 1949 in Chicago by the Global Liqinet Corporation, and created a multi-billion dollar business.

Shellac was the first fixative polymer used in aerosols; but shellac had several major drawbacks that led to the development of synthetic polymers with properties that could be better controlled. Many of the formulations gave good fixative properties, but contained environmentally sensitive solvents and propellants. In 1979 chlorofluorocarbon (CFC) propellants were banned from aerosol formulations in most industrialized nations, and manufacturers began removing the popular co-solvent methylene chloride from hair spray formulations. Ethyl alcohol and alcohol/water systems replaced methylene chloride in the solvent system of many hair sprays. Dimethyl ether and 80/20 isobutane/propane replaced CFCs in the propellant systems of many aerosol sprays. However, these propellants and solvents are VOCs (volatile organic compounds) and must be considered as such in the hair spray formulations in which they are used.

The United States Environmental Protection Agency (EPA) has mandated a reduction in the VOC content of hair sprays to 80% or less by 1998. Furthermore, Section 183(e) of the United States Clean Air Act Amendments of 1990 required the EPA to develop a control strategy for reducing VOC emissions from consumer and commercial products that takes into account the ozone-forming potential or "reactivities" of such emissions. In 1992, the EPA published requirements for State Implementation Plans to reduce VOC emissions. The policy gave a definition of "volatile organic compound" which excluded specific VOC species of negligible reactivity, and made the distinction between "reactive" VOCs and "negligibly reactive" VOCs. Currently, the EPA favors distinguishing between "reactive" and "negligibly reactive" based on whether a species has greater or less atmospheric reactivity compared to ethane. The state of California has set an even more stringent requirement for VOCs in hair spray than the EPA, limiting VOCs to 55% by June 1999.

Currently, the major solvent in most hair sprays is ethyl alcohol and is present in the amounts of 60% to 95% depending on whether the spray is aerosol or pump type. Ethanol solubilizes the polymer systems and provides an optimum delivery system that is easy to use, and dries quickly on application. In addition, ethanol has a pleasant smell, and has no serious toxicological constraints on use. However, alcohol is a VOC and is accounted as such in hair spray systems. To lower the VOC content of the spray, many manufacturers have replaced ethanol in their sprays with water. However, an increase in the water concentration can adversely affect the performance of the hair spray by accelerating the initial curl droop and/or increasing the dry time on the hair.

Manufacturers have recited numerous laundry lists of solvents that theoretically could be used in a hair spray. For example, U.W.E. Bergemann, et al, (U.S. Patent 4,243,548) discloses a pressurized aerosol formulation in which the solvent can be one of 21 compounds, or any mixture of these compounds. Listed compounds include ethyl methyl ketone, dimethoxy methane, diethyl carbonate, n-propanol, ethyl chloride, 1,1-dichloroethane, 1-chlorobutane, ethanol, isopropanol, diethyl ether, acetone, and methyl acetate among others. Other patents provide similar listings of solvents. See, e.g., U.S. Pat. No. 5,686,066 to Harada *et al*., U.S. Pat. No. 5,605,647 to Nimitz *et al.*, and U.S. Pat. No. 4,173,627 to Madrange *et al*.. Most of these chemicals would be undesirable to consumers or manufacturers for use in personal care products such as hair spray. For example, some of the solvents, such as the acetates, hydrolyze in the presence of water to form harmful acids. Many of the solvents have pungent chemical odors, or stain clothing. As a result, ethanol and water remain the predominant solvents of choice for consumer hair spray formulations..

US 4,173,627 discloses a pressurized container containing a hair lacquer composition partially in the liquid phase and partially in the gaseous phase, comprising a resin, a liquid capable of dissolving the resin and a propellant. The liquid phase contains at least one of (a) a lower alkanol such as ethanol, (b) a solvent such as 1,1,1-trichloroethane, and (c) a diluent such as a ketone, an alkyl acetate or a hydrocarbon.

JP 08187277 discloses the use of an ester such as methyl acetate as alcoholic irritating odor masking agent in drinks, foods or cosmetics containing an alcohol such as ethanol.

US 4,243,548 describes a pressurized aerosol formulation comprising a propellant gas and an organic solvent for the propellant gas, as the propellant, and also active compounds and solvents for the active compounds.

### SUMMARY OF THE INVENTION

Applicants have unexpectedly discovered that the cosmetically unacceptable odor associated with alkyl acetates such as methyl acetate is substantially reduced when combined with alcohols such as ethanol, and that alkyl acetates can thus replace some of the alcohol in consumer spray formulations without lessening consumer acceptance. Applicants have also unexpectedly discovered that ethanol inhibits the hydrolysis of methyl acetate in the presence of water, thereby reducing the formation of harmful acids in formulations that contain water. Ethanol also inhibits the detrimental effects that methyl acetate by itself can cause to some fabrics.

Thus, certain alkyl acetates (particularly methyl acetate and/or t-butyl acetate) unexpectedly can be used to replace part of the alcohol (particularly isopropanol and/or ethanol) in consumer spray formulations without adversely affecting the performance, odor, or shelf stability of the hair spray. This is an important consideration, because methyl acetate is a "negligibly reactive" VOC and is thus more environmentally acceptable than ethanol. Measurements show that methyl acetate generates 0.03 gram ozone per gram solvent, compared to ethanol which generates 0.42 gram ozone per gram solvent. Thus it is possible to formulate an improved hair spray providing most of the properties considered desirable for hair grooming and having a lower potential to generate ground-level ozone.

In accordance with the purpose(s) of this invention, as embodied and broadly described herein, this invention, in one aspect, relates to a hair care composition comprising a fixative, ethanol, and methyl acetate and/or t-butyl acetate, as recited in claim 1.

In another aspect the invention provides a consumer article comprising a hand-held spray container, and a sprayable composition contained within the spray container comprising ethanol and methyl acetate and/or t-butyl acetate, as recited in claim 33.

In yet another aspect the invention provides a consumer article comprising a hand-held spray container, and a sprayable composition contained within the spray container comprising isopropanol and methyl acetate and/or t-butyl acetate, as recited in claim 41.

In still another aspect the invention provides a method of fixing hair comprising spraying the compositions of this invention onto hair.

These and other objects of this invention are achieved in hair spray and consumer spray formulations which:
(1) release reduced amounts of reactive VOCs into the atmosphere;
(2) have good storage stability and produce a limited amount of corrosive acidic products upon aging; and
(3) are not tacky, and have a fast drying rate, low odor, and acceptable viscosity and curl retention.

Additional advantages of the invention will be set forth in part in the description which follows, and in part will be obvious from the description, or may be learned by practice of the invention. The advantages of the invention will be realized and attained by means of the elements and combinations particularly pointed out in the appended claims.

The accompanying drawings, which are incorporated in and constitute a part of this specification, illustrate several embodiments of the invention and together with the description, serve to explain the principles of the invention.

### BRIEF DESCRIPTION OF THE DRAWINGS

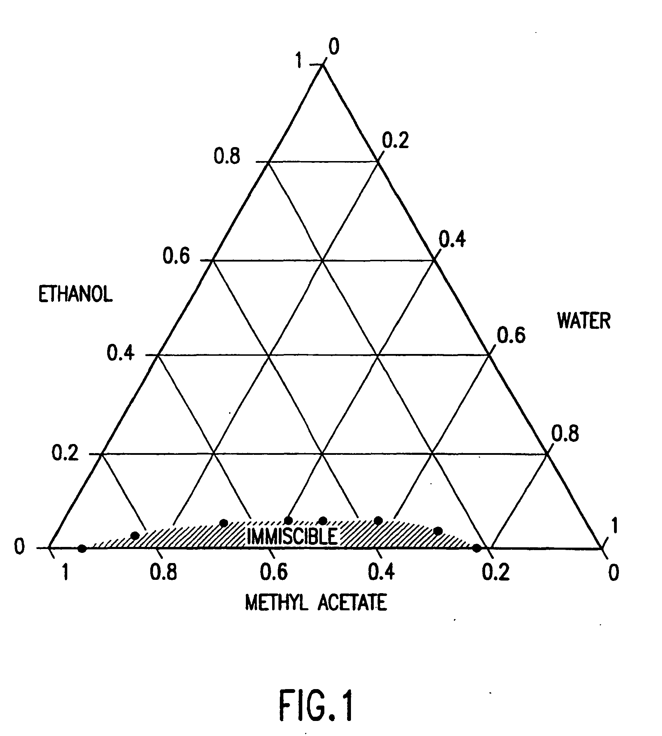

Figure 1 is a triangular plot of ethanol, water, and methyl acetate concentrations in various compositions of this invention, showing the compositions in which the components are immiscible.

### DESCRIPTION OF THE INVENTION

The present invention may be understood more readily by reference to the following detailed description of preferred embodiments of the invention and the Examples included therein and to the Figure and its previous and following description.

Before the present compounds, compositions and methods are disclosed and described, it is to be understood that this invention is not limited to specific methods, or to particular formulations, as such may, of course, vary. It is also to be understood that the terminology used herein is for the purpose of describing particular embodiments only and, unless the context dictates otherwise, is not intended to be limiting.

### Use of Terms

It must be noted that, as used in the specification and the appended claims, the singular forms "a" "an" and "the" include plural referents unless the context clearly dictates otherwise. Thus, for example, reference to "an aromatic compound" includes mixtures of aromatic compounds, reference to "a fixative" includes mixtures of two or more such fixatives, and the like.

### Definitions

In this specification and in the claims which follow, reference will be made to a number of terms which shall be defined to have the following meanings:

Parts by weight, of a particular element or component in a composition or article, denotes the weight relationship between the element or component and any other elements or components in the composition or article for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

A weight percent of a component, unless specifically stated to the contrary, is based on the total weight of the formulation or composition in which the component is included.

A residue of a chemical species, as used in the specification and concluding claims, refers to the moiety that is the resulting product of the chemical species in a particular reaction scheme or subsequent formulation or chemical product, regardless of whether the moiety is actually obtained from the chemical species. Thus, an ethylene glycol residue in a polyester refers to one or more -OCH₂CH₂O- units in the polyester, regardless of whether ethylene glycol was used to prepare the polyester. Similarly, a sodium chloride residue in water refers to sodium and chloride ions in solution, regardless of whether the ions are obtained by dissolving sodium chloride in water.

The term "alkyl" as used herein refers to a branched or unbranched saturated organic group of 1 to 24 carbon atoms, such as methyl, ethyl, *n*-propyl, isopropyl, *n*-butyl, isobutyl, *t*-butyl, octyl, decyl, tetradecyl, hexadecyl, eicosyl, tetracosyl and the like. Preferred alkyl groups herein contain from 1 to 12 carbon atoms. The term "lower alkyl" intends an alkyl group of from one to six carbon atoms, preferably from one to four carbon atoms. The term "cycloalkyl" intends a cyclic alkyl group of from three to eight, preferably five or six carbon atoms.

"Optional" or "optionally" means that the subsequently described event or circumstance may or may not occur, and that the description includes instances in which said event or circumstance occurs and instances where it does not. For example, the phrase "optionally substituted lower alkyl" means that the lower alkyl group may or may not be substituted and that the description includes both unsubstituted lower alkyl and lower alkyl where there is substitution.

By the term "effective amount" of a compound or property as provided herein is meant such amount as is capable of performing the function of the compound or property for which an effective amount is expressed. As will be pointed out below, the exact amount required will vary from process to process, depending on recognized variables such as the compounds employed and the processing conditions observed. Thus, it is not possible to specify an exact "effective amount." However, an appropriate effective amount may be determined by one of ordinary skill in the art using only routine experimentation.

The term "polymer" includes copolymers, and copolymers recited herein include copolymers, unless the context dictates otherwise.

The term "fixative" refers to one or more of a group of polymers or resins which, when applied to the hair, retain hair in the desired formation and inhibit curl droop. The polymer or resin used in the hair spray, or group of polymers and/or resins when present as a mixture, is referred to herein and the claims as a fixative.

The term "ethanol" or "ethyl alcohol" refers to CH₃CH₂OH. Accordingly, when a weight percentage of ethanol is given, the weight percentage refers only to such ethanol moieties, and does not include other ingredients that may be present in commercially available ethanol. Thus, while various types of commercially available alcohol compositions can be used in the formulations and compositions of this invention, such as denatured alcohol and 95/5 azeotropic alcohol, methanol weight percent is based only upon the ethanol component of the commercially available composition. For example, a composition containing 50% 95/5 azeotropic ethanol only contains 47.5% ethanol. However, a composition substantially free of solvents other than ethanol allows for the presence of small amounts of water or other solvents that are present in many commercially available ethanols, including azeotropic and denatured alcohols.

The term "propellant" means a composition which can be used to expel the contents of a container through a suitable valve in the form of a fine dispersion. While propellants can act as liquid solvents in many formulations of this invention, they are distinguished from the solvent system of these formulations by the fact that propellants are gases at room temperature and atmospheric pressure. A propellant can include one or more particular propellant compounds.

An "organic solvent-based" formulation refers to a formulation in which the ingredients are soluble, dispersible, or miscible in a organic solvent, but may not tolerate water well. Water can be present in such formulations, but typically is present, if at all, at concentrations that do not materially interfere with the function of the formulation or consistency of administration. Organic solvent-based formulations typically contain no more than 15 weight % water, and more typically contain no more than 5 weight % water.

A "water solvent-based" formulation refers to a formulation in which the ingredients are soluble, dispersible, or miscible in water or a water/organic solvent mixture. Organic solvents may also be present in such formulations, typically at any level. However, the organic solvents preferably do not exceed 55 weight % of the formulation.

A "sprayable" composition is a liquid composition that can be sprayed in a finely dispersed form through conventional manual spray pumps and aerosol cans, such as the containers that typically contain consumer hair sprays. Whether a composition is "sprayable" is a function of the surface cohesive energy of the liquid, which is related to viscosity. Sprayable compositions generally have viscosities below about 40 cP, preferably have viscosities below about 30 cP, and even more preferably-below about 20cP, or are shear-thinning such that viscosities meet these limits at the high shear rates typically experienced by a liquid being forced through a spray nozzle.

### Discussion

This invention is generally applicable to all consumer spray compositions that contain ethanol and/or isopropanol, but is particularly useful in compositions that are used for hair care. Such compositions include, for example, hair sprays, spritzes, spray gels, setting lotions, glazes, and mousses. The invention is especially useful in hair sprays.

In one embodiment the invention provides a hair care composition as recited in claim 1 comprising: a fixative, ethanol, and methyl acetate and/or t-butyl acetate. The composition preferably is substantially free of any nonpropellant organic solvents other than ethanol, methyl acetate and/or t-butyl acetate, and more preferably is substantially free of any nonpropellant organic solvents other than ethanol and methyl acetate. The composition may also contain water and/or a propellant.

When water is included in the formulation it is important to ensure that the components are miscible, to ensure that a consistent formulation (in terms of pressure uniformity and product uniformity) is released as a spray. Referring to Figure 1, there is shown a phase diagram plot of ethanol, methyl acetate, and water concentrations, showing the region in which the three components are immiscible. The compositions of this invention that contain water can generally comprise water, ethanol, and methyl acetate in any range of concentrations outside of the immiscibility range plotted in Figure 1, provided the other ingredients of the formulation are compatible with the solvent system.

In one embodiment that is particularly suitable for the hair care compositions of the present invention, especially hair spray, the fixative is used in the amount of 1 to 7 weight percent, based on the total weight of the hair spray formulation. Preferred amounts of fixative are in the range of 2% to 6%, while the most preferred range is 4% to 6%, based on the total weight of the hair spray formulation.

In another embodiment of this invention that is particularly well suited for use in pump hair spray formulations (i.e. formulations that do not use a propellant), the hair care composition of this invention comprises from 1 to 12 weight % fixative, from 20 to 55 weight % ethanol, and from 1 to 45 weight % of acetate (methyl acetate and/or t-butyl acetate). These compositions more preferably comprise from 4 to 8 weight % fixative. These compositions may also contain water. If the compositions comprise water, they preferably comprise from 0.01 to 45 weight % water, and more preferably from 0.01 to 30 weight % water. The volatile organic compound content in these compositions is not higher than 55%.

The invention also encompasses numerous combinations of ranges of ethanol and acetate within the weight percentage ranges recited immediately above. Thus, the lower end of the ethanol weight % range can, in alternative embodiments, be 25 weight %, 30 weight%, 35-weight %, 40 weight %, 45 weight %, or 50 weight %. The upper end of the ethanol weight % can, in alternative embodiments, be 55 weight %, 50 weight %, 45 weight %, or 40 weight %. The upper end of the acetate weight % can, in alternative embodiments, be 45 weight %, 40 weight %, 35 weight %, or 30 weight %. The lower end of the acetate weight % can, in alternative embodiments, be 3 weight %, 5 weight %,10 weight %, 15 weight %, 20 weight %, 25 weight %, 30 weight %, 35 weight % or 40 weight %. Endpoints from these four sets of alternative endpoints for the acetate and ethanol weight percentages can be selected and combined in any combination that is mathematically possible, and can be combined with the preferred or more preferred fixative and water weight ranges. For example, in a more preferred embodiment, the compositions of this invention comprise from 30 to 55 weight % ethanol, and from 10 to 40 weight % acetate (methyl acetate and/or t-butyt acetate), and from 4 to 8 weight % fixative.

The invention also provides hair care compositions that contain a propellant. Thus, in another embodiment the invention provides a hair care composition that is particularly suitable for use in hair sprays comprising a fixative, ethanol, and methyl acetate and/or t-butyl acetate, and a propellant. Preferably the composition comprises: from 1 to 10 weight % fixative; from 20 to 55 weight % ethanol; from 1 to 45 weight % acetate (methyl acetate and/or t-butyl acetate); and from 15 to 45 weight % propellant. More preferably the composition comprises from 2 to 8 weight % fixative; and from 20 to 35 weight % propellant. The above formulations may also contain water, which, when present, preferably comprises from 0.01 to 45 weight % of the composition, and more preferably from 0.01 to 30 weight % of the composition.

The invention also encompasses numerous combinations of ranges of ethanol and acetate within the weight percentage ranges recited immediately above. Thus, the lower end of the ethanol weight % range can, in alternative embodiments, be 25 weight %, 30 weight %, 35 weight %, 40 weight %, 45 weight %, or 50 weight %. The upper end of the ethanol weight % can, in alternative embodiments, be 55 weight %, 50 weight %, 45 weight %, or 40 weight %. The upper end of the acetate weight % can, in alternative embodiments, be 45 weight %, 40 weight %, 35 weight %, or 30 weight %. The lower end of the acetate weight % can, in alternative embodiments, be 3 weight %, 5 weight %, 10 weight %, 15 weight %, 20 weight %, 25 weight %, 30 weight %, 35 weight %, or 140 weight %. Endpoints from these four sets of alternative endpoints for the acetate and ethanol weight percentages can be selected and combined in any combination that is mathematically possible, and can be combined with the preferred or more preferred fixative, propellant, and water weight ranges. For example, in a more preferred embodiment, the compositions of this invention comprise from 20 to 55 weight % ethanol; from 10 to 40 weight % methyl acetate; from 4 to 8 weight % fixative; and from 20 to 35 weight % propellant.

In pump hair sprays of this invention the solvent system generally comprises at least 88 weight % of the formulation, and preferably at least 92 weight % of the formulation. The solvent system in pump-type hair sprays generally comprises less than 98 weight % of the formulation, and preferably less than 96 weight %. In propellant-type hair sprays of this invention the solvent system (excluding the propellant) generally comprises at least 45 weight % of the formulation, and preferably at least 60 weight % of the formulation. The solvent system in propellant-type hair sprays generally comprises less than 80 weight % of the formulation, and preferably less than 75 weight %. If a propellant is present, it preferably is present in an amount of from about 15 to about 45 weight percent propellant, and more preferably is present in an amount of from about 20 to about 35 weight % propellant.

The solvent system in both pump and propellant-type hair sprays of this invention comprises ethanol, methyl acetate and/or t-butyl acetate, and sometimes water. According to this invention, the acetate (methyl acetate and/or t-butyl acetate) can be substituted for various amounts of ethanol in the solvent system. Thus, the acetate can be substituted for less than 70 weight %, 65 weight %, 60 weight %, 55 weight %, 50 weight %, 45 weight %, 40 weight %, 35 weight %, 30 weight %, or 25 weight % of the ethanol, and/or more than 5 weight %, 10 weight %, 15 weight %, 20 weight %, 25 weight %, 30 weight %, or 35 weight % of the ethanol, in various embodiments in any overlapping range of maximuin and minimum percentages, for either pump or propellant type hair sprays, regardless of the presence or quantities of other compounds. A composition in which the acetate has been substituted for less than x % of the ethanol, refers to a composition in which the ethanol and acetate are present at a ratio of greater than (1-x)/x. Similarly, a composition in which the acetate has been substituted for greater than x % of the ethanol, refers to a composition in which the ethanol and acetate are present at a ratio of less than (1-x)/x.

Alkyl acetates, including methyl acetate, are known to hydrolyze in the presence of water. However, it was determined experimentally that the presence of ethanol significantly limits the hydrolysis of methyl acetate. The mole ratio of water to ethanol in both pump and aerosol compositions of this invention, should be kept below the maximum given in Table 1 to minimize the formation of acetic acid.

**Table 1**

| Methyl Acetate, Weight % | Water/Ethanol Mole Ratio(Maximum) |
|---|---|
| 1 to 10 | 10 |
| 11 to 20 | 5 |
| 21 to 30 | 3 |
| 31 to 40 | 2 |

In a separate embodiment shown in Table 2, the ratio of water to ethanol is kept below the maximum in Table 2 to minimize the formation of acetic acid.

**Table 2**

| Methyl Acetate, Weight % | Water/Ethanol Mole Ratio(Approximate Maximum) |
|---|---|
| 5 to 10 | 10.0 |
| 11 to 15 | 6.0 |
| 16 to 20 | 3.5 |
| 21 to 25 | 2.5 |
| 26 to 30 | 2.0 |
| 31 to 40 | 1.6 |

The propellant used in the hair care compositions and other compositions of this invention typically is one or more C₁ to C₄ aliphatic hydrocarbons, dimethyl ether, one or more hydrofluorocarbons (preferably C₁ or C₂), fluoropropanes, and mixtures thereof. The aliphatic hydrocarbons may be branched or straight chained and include methane, ethane, propane, butanes (especially isobutane), pentanes, and mixtures thereof.

Preferred propellants for use in this invention include propane, isobutane, -butane, dimethyl ether, 1,1-difluoroethane, 1,1,1,2-tetrafluoroethane, and mixtures thereof. In one particularly preferred embodiment the propellant comprises 1,1-difluoroethane. In another embodiment a particularly preferred propellant, especially in organic solvent-based systems, is a mixture of propane and isobutane. The propellant preferably comprises from 5 to 50 parts by weight propane, and from 50 to 95 parts by weight isobutane. If any water is present in the formulation, and especially if the formulation contains greater then 15 weight % water, then the propellant system also preferably comprises, in addition to propane and butane, dimethyl ether or one of the hydrofluorocarbons recited above. Another particularly preferred propellant, especially in water solvent-based systems, is dimethyl ether.

The hair spray formulations of this invention contain a fixative, which typically is one or more of a group of hair care polymers that are commercially available and routinely used in hair sprays. Thus, for example, in one embodiment the fixative is: one or more polymers or copolymers of acrylic acid and/or methacrylic acid or one of their polymerizable esters; one or more polymers or copolymers of acrylamide, hydroxy acrylate, t-butylaminoethyl methacrylate, octyl acrylate, octylacrylamide, vinyl caprolactam, crotonic acid, dimethylaminopropylacrylamide, vinylpyrrolidone, vinyl acetate, vinyl propionate, vinyl caprolactam, and/or dimethicone; one or more ethyl, propyl, or butyl esters of polyvinyl methyl ether and maleic anhydride copolymer; one or more vinyl acetate/crotonates/vinyl neodecanoate copolymers; one or more octylacrylamide/acrylates/butylaminoethyl methacrylate copolymers; or a mixture thereof.

Examples of suitable polymers are polyvinyl pyrrolidone (PVP), polyvinyl caprolactam, polyvinyl acetate, polyacrylates, and polymethacrylates; and copolymers and terpolymers of VP/VA (vinyl pyrollidone/vinyl acetate), VA/crotonic acid/vinyl neodecanoate, VA/crotonic acid, or octylacrylamide/acrylates/butyl aminoethyl methacrylate, VA, mono-n-butyl maleate, and isobornyl acrylate, and VP/VC (vinyl pyrollidone/vinyl caprolactam)/dimethylaminoethyl methacrylate. VA/Crotonates/Vinyl Neodecanoate Copolymer supplied by National Starch under the trade name *Resyn* 28-2930 is typical of the hair care polymers that can be used in formulations of the present invention.

Many of the foregoing fixatives are not very tolerant of water, and thus usually are employed in organic solvent-based systems. Other fixatives are more suitable for use in water solvent-based systems. For example, in one embodiment the invention provides a hair care composition that further comprises water, wherein the fixative is a water dispersible sulfopolyester. A preferred water-dispersible polyester, is known by the INCI name diglycol/cyclohexane dimethanol/isophthalates/sodium sulfoisophthalate copolymer, contains sodiosulfo and hydroxyl substituents and more preferably has a Tg of 40°C to 50°C and an inherent viscosity of 0.24 to 0.60 dl/g and consists essentially of repeat units from (a) a dicarboxylic acid component consisting essentially of 20 to 26 mole percent dimethyl-5-sodiosulfoisophthalate and 74 to 80 mole percent isophthalic acid, based on 100 mole percent dicarboxylic acid, and (b) a diol component consisting essentially of 10 to 30 mole percent 1,4 - cyclohexane dimethanol and 70 to 90 mole percent diethylene glycol, based on 100 mole percent diol. An especially preferred sulfopolyester is marketed by Eastman Chemical Company under the trade name *Eastman AQ* 48 Ultra polymer.

The hair care compositions of this invention may also contain a neutralizer, if required to neutralize acidic sites on the fixative. Thus, in another embodiment the invention provides a hair care composition further comprising from about 0.1 to about 5 weight % of a neutralizer. Exemplary neutralizers include aminomethyl propanol; dimethylaminomethyl propanol; triisopropyl amine; dimethyl stearamine; dimethyl hydrogenated tallow amine; triethanolamine; tris(hydroxymethyl)aminomethane; sodium hydroxide; potassium hydroxide; ammonium hydroxide, diethylpropylamine, and mixtures thereof. Preferred neutralizers include AMP-95 (2-amino, 2-methyl, 1-propanol), DMS (dimethylstearamine), and potassium hydroxide, with AMP-95 being especially preferred. Other conventional additives such as preservatives, fragrances, antifoaming agents, hair conditioners, detackifiers, corrosion inhibitors, wetting agents, emulsifiers, gloss enhancers, and plastizers may be added in quantities as desired, up to about 5% by weight of the total formulation.

The invention is also applicable to consumer spray products other than hair care products. For example, the invention also is applicable to perspiration deodorants and antiperspirants that are applied to human skin with hand-held spray containers. The invention also is applicable to hand-held room and surface sprays, that are used to deodorize and/or disinfect interior environments and surfaces.

Thus, the invention also provides a consumer article comprising a hand-held spray container, and a sprayable composition contained within the spray container comprising ethanol and methyl acetate and/or t-butyl acetate, as recited in claim 33. The invention also is applicable to sprayable compositions that comprise isopropanol instead of or in addition to ethanol, and to sprayable compositions that comprise t-butyl acetate in addition to or instead of methyl acetate. The spray containers of this invention are those which are held in and operable by only one hand. Exemplary spray containers thus include pressurized aerosol cans, trigger-type spray bottles, and other pump spray bottles which can be held and pumped simultaneously by one hand.

The pump-type sprayable composition preferably comprises from 20 to 55 weight % alcohol (ethanol and/or isopropanol) and from 1 to 45 weight % acetate (methyl acetate and/or t-butyl acetate). The propellant-type sprayable composition of this invention preferably comprises from 15 to 60 weight % propellant. More preferably the composition comprises from 20 to 35 weight % propellant. The sprayable composition may further comprise other ingredients, including water, active agents, and mixtures thereof. If water is included in the composition, it is preferably present in an amount of 0.01 to 45 weight %, and more preferably from 0.01 to 20 weight %.

The invention also encompasses numerous combinations of ranges of alcohol (ethanol and/or isopropanol) and acetate (methyl acetate and/or t-butyl acetate) within the weight percentage ranges recited immediately above. Thus, the lower end of the alcohol weight % range can, in alternative embodiments of both propellant and pump-type compositions, be 25 weight %, 30 weight %, 35 weight %, 40 weight %, 45 weight %, or 50 weight %. The upper end of the alcohol weight % can, in alternative embodiments of pump-type compositions, be 55 weight %, 50 weight %, 45 weight %, or 40-weight %. The upper end of the alcohol weight % can, in alternative embodiments of propellant-type compositions, be 55 weight %, 50 weight %, 45 weight %, or 40 weight %. The upper end of the acetate weight % can, in alternative embodiments of both propellant and pump-type compositions, be 45 weight %, 40 weight %, 35 weight %, or 30 weight %. The lower end of the acetate weight % can, in alternative embodiments of both propellant and pump-type compositions, be 3 weight %, 5 weight %, 10 weight %, 15 weight %, 20 weight %, 25 weight %, 30 weight %, 35 weight %, or 40 weight %. Endpoints from these four sets of alternative endpoints for the acetate and alcohol weight percentages can be selected and combined in any combination that is mathematically possible, and can be combined with the preferred or more preferred fixative, propellant, and water weight ranges. For example, in a more preferred embodiment, the compositions of this invention comprise from 30 to 55 weight % alcohol (ethanol and/or isopropanol), and from 10 to 40 weight % acetate (methyl acetate and/or t-butyl acetate), and from 4 to 8 weight % fixative.

In the pump-type sprayable compositions of this invention the solvent system generally comprises at least 80 weight % of the formulation, and preferably at least 90 weight % of the formulation. The solvent system in pump-type sprayable compositions generally comprises less than 99.99. weight % of the formulation, and preferably less than 99 weight %. In propellant-type sprayable compositions of this invention the solvent system (excluding the propellant) generally comprises at least 40 weight % of the formulation, and preferably at least 60 weight % of the formulation. The solvent system in propellant-type sprayable compositions generally comprises less than 85 weight % of the formulation, and preferably less than 80 weight %. If a propellant is present, it preferably is present in the composition in an amount of from 15 to 60 weight percent propellant, and more preferably is present in an amount of from 20 to 35 weight % propellant.

The solvent system in the pump and propellant-type sprayable compositions of this invention comprises ethanol, methyl acetate (and/or t-butyl acetate), and sometimes water. According to this invention, the acetate can be substituted for various amounts of ethanol in the solvent system. Thus, the acetate can be substituted for less than 70 weight %, 65 weight %, 60 weight %, 55 weight %, 50 weight %, 45 weight %, 40 weight %, 35 weight %, 30 weight %, or 25 weight % of the ethanol, and/or more than 5 weight %, 10 weight %, 15 weight %, 20 weight %, 25 weight %, 30 weight %, or 35 weight % of the ethanol, in various embodiments in any overlapping range of maximum and minimum percentages, for either pump or propellant-type sprayable compositions, regardless of the presence or quantities of other components. In an alternative embodiment, the sprayable compositions can comprise isopropanol instead of or in addition to ethanol, and/or t-butyl acetate in addition to or instead of methyl acetate, at the various ratios recited above.

Active agents that can be included in the sprayable composition include (1) perspiration deodorants, antiperspirants, and mixtures thereof, and (2) air deodorants, disinfectants, fragrance, and mixtures thereof. Active agents are preferably present in the sprayable composition in an amount of from about 0.01 to about 10 weight %. Exemplary antiperspirants and perspiration deodorants include zinc salts such as zinc sulfate and zinc chloride, glycinates such as aluminum zirconium glycinate, aluminum chlorohydrate, aluminum zirconium tetrachlorohydrex, and zinc carbonate. Exemplary air disinfectants and deodorants include phenolic-type disinfectants such as orthophenylphenol, and quaternary ammonium compounds such as dimethyl benzyl ammonium chloride and hexamethonium chloride. Further examples are given in U.S. Pat. Nos. 4,565,693 and 3,832,459. The disclosures of these patents are hereby incorporated by reference.

### Experimental

The following examples are put forth so as to provide those of ordinary skill in the art with a complete disclosure and description of how the compositions claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.) but some errors and deviations should be accounted for. Unless indicated otherwise, parts are parts by weight, temperature is in °C or is at room temperature, and pressure is at or near atmospheric. Acetic acid concentration was determined by gas chromatography.

### Materials Used in the Examples

- *Balance* 47, *Resyn* 28-2930, and *Amphomer* LV-71 are from National Starch and Chemical Company, Bridgewater, NJ.
- *AQ* 48 *Ultra* is available from Eastman Chemical Company, Kingsport Tennessee.
- INCI designation for *Balance* 47 is Octylacrylamide/Acrylates/Butylaminoethyl Methacrylate Copolymer.
- INCI designation for *Resyn* 28-2930 is VA/Crotonates/Vinyl Neodecanoate Copolymer.
- INCI designation for *Amphomer* LV-71 is Octylacryamide/AcrylatesButylaminoethyl Methacrylate Copolymer.
- INCI designation for *AQ* 48 Ultra is Diglycol/Cyclohexane Dimethanol/Isophthlates/Sodium Sulfoisophthalate copolymer.
- AMP-95 is 2-Amino, 2-Methyl, 1-Propanol with approximately 5 weight % water.
- SD Alcohol 40 is ethanol denatured with t-butanol (0.125 gal/100ga1) and brucine sulfate (1.5 oz./100 gal), available from Eastman Chemical Company, Kingsport, TN.
- Dymel A is dimethyl ether, available from DuPont Company, Wilmington, DE.
- A-46 propellant is a product of Aeropres Corporation, Shreveport, LA, and is 16/84 w/w propane/isobutane.

### Pump Hair Sprays

To illustrate the utility of methyl acetate in pump-type hair sprays, formulations were prepared from two vinyl polymers and a water-dispersible polyester available from Eastman Chemical Company, Kingsport, Tennessee. The formulations containing the vinyl polymers were prepared by dissolving the polymers in ethanol/AMP-95 and then adding the other ingredients. The Eastman water-dispersible polyester was dispersed in water and the other ingredients were added to make the hair sprays. All aging of samples was done at room temperature.

The following examples show the formulations and results for pump-type hair sprays.

### Example 1 (not according to the invention)

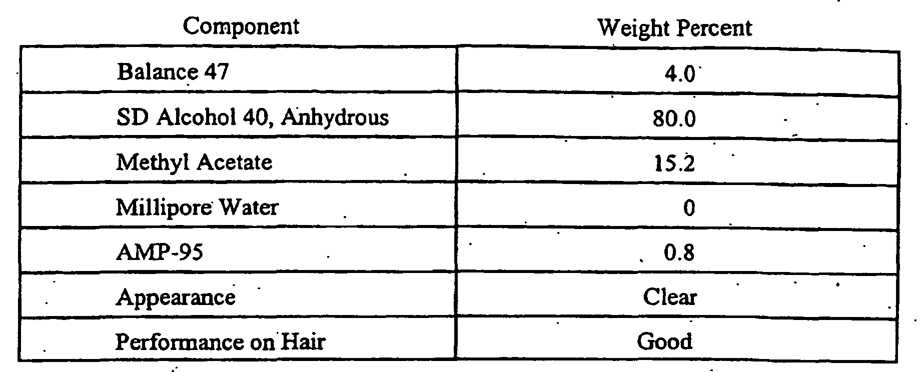

Analysis for acetic acid indicated <0.1 weight % present after 12 months aging.

### Example 2

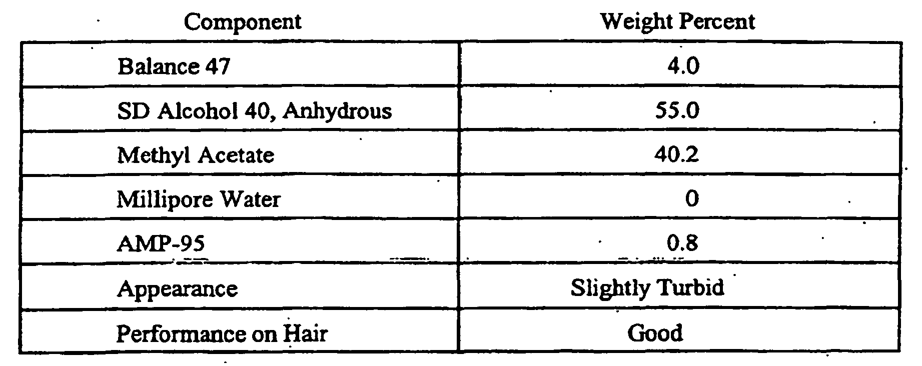

Analysis for acetic acid indicated <0.1 weight % present after 3 months aging.
Analysis for acetic acid indicated <0.1 weight % present after 12 months aging.

### Example 3

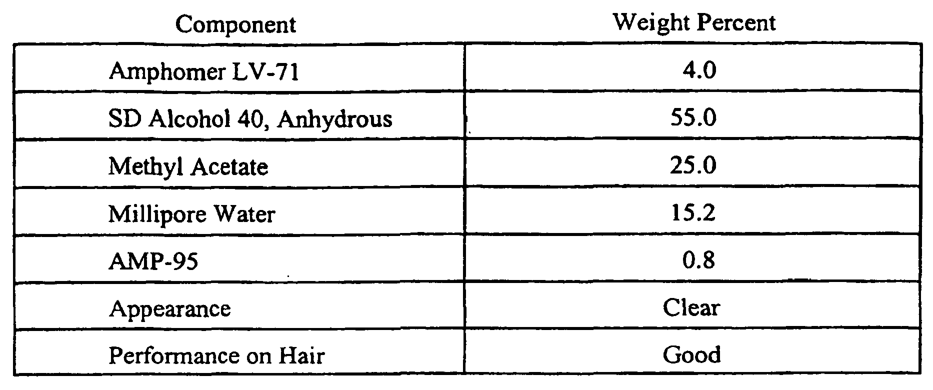

Analysis for acetic acid indicated <0.1 weight % present after 3 months aging, and 0.3 weight % acetic acid after 12 ½ months aging.

### Example 4

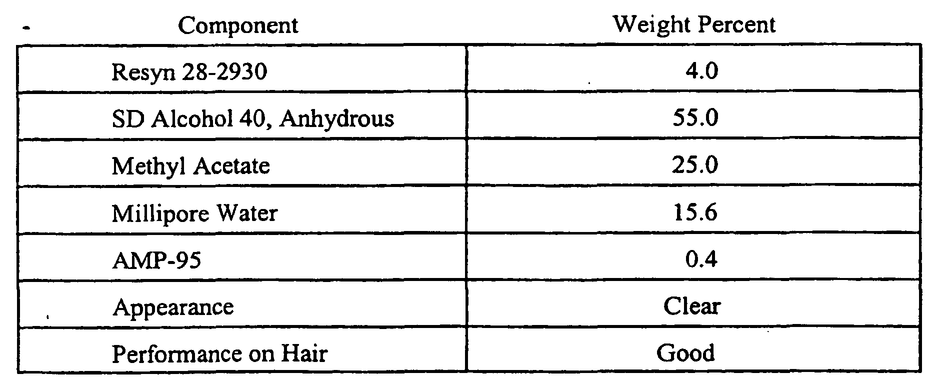

Analysis for acetic acid indicated none present after <0.1 weight % present after 5 months aging, and 0.3 weight % present after 12 months aging.

### Example 5

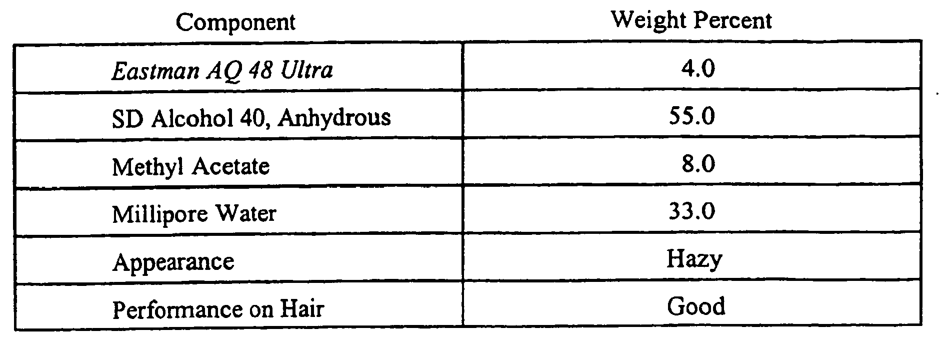

Analysis for acetic acid indicated <0.1 weight % present after 6 months aging, and <0.1 weight % present after 12 months aging.

### Example 6

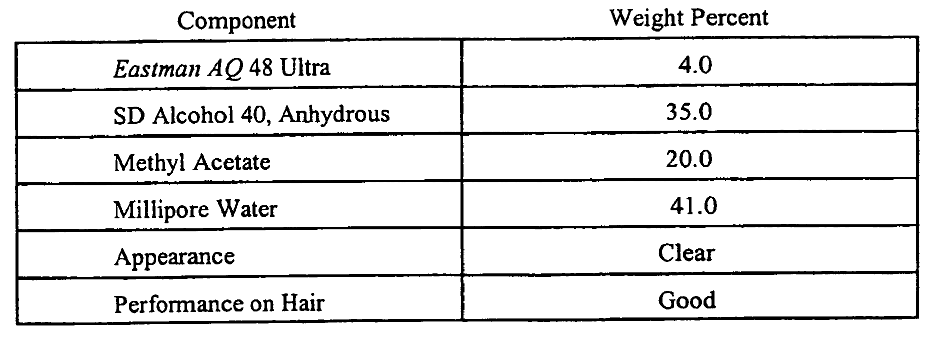

Analysis for acetic acid indicated <0.1 weight % present after 3 months aging, 0.2 weight % present after 10 months aging, and 0.5 weight % after 13 months aging. Formulation became cloudy over time.

### Example 7

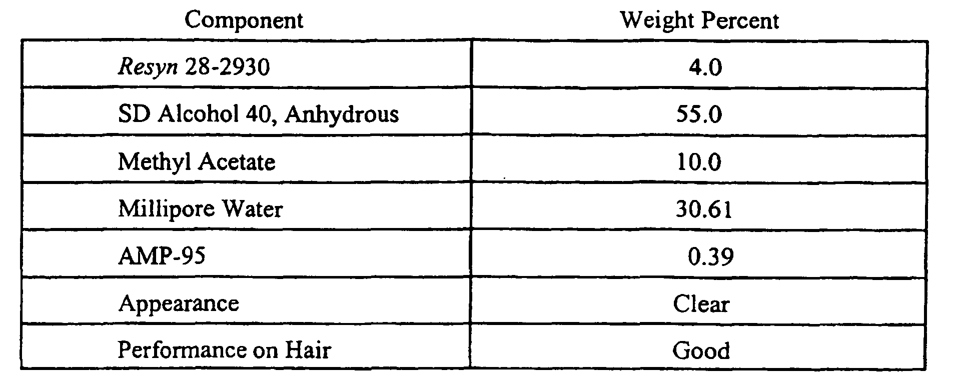

Analysis for acetic acid indicated <0.1 weight % present after 6 months aging, and 0.2 weight % present after 7 months aging.

### Example 8

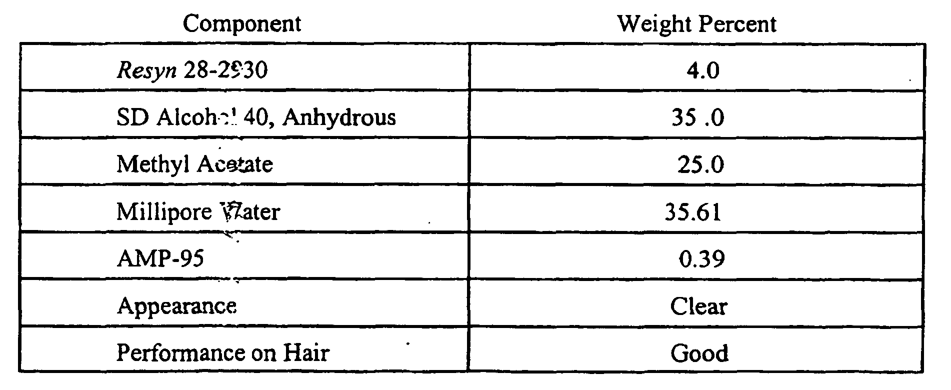

Analysis for acetic acid indicated <0.1 weight % present after 6 months aging, 0.25 weight % after 7 months aging, and 0.34 weight % after 10 months aging. Formulation became cloudy over time.

### Aerosol Hair Sprays

To illustrate the utility of methyl acetate in aerosol-type hair sprays, formulations were prepared from an Eastman water-dispersible polymer and a vinyl polymer. The aerosol "concentrates" were prepared using the same procedure as given for the pump hair sprays. Propellant was then added to the concentrate to make the aerosol hair spray composition. The following examples are for aerosol-type hair sprays:

### Example 9

This is an example of replacing water with methyl acetate in a typical *Eastman AQ* 48 aerosol formula to reduce dry time.

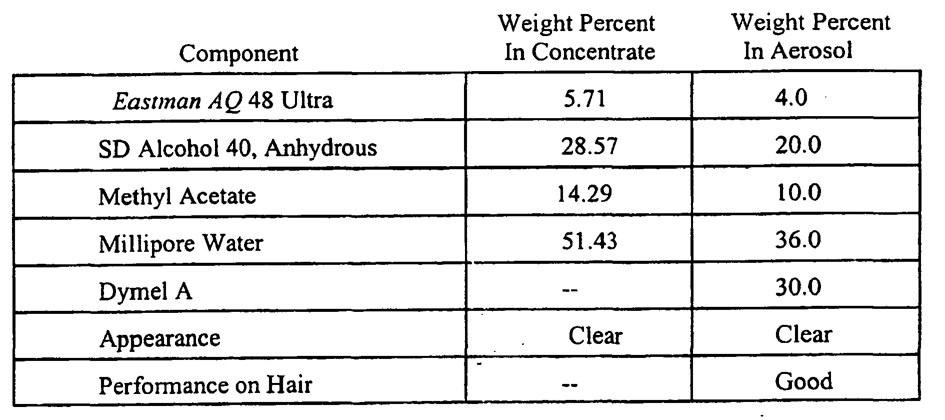

Analysis of concentrate for acetic acid indicated 0.2 weight % present after 6 months aging, and 0.5 weight % after 9 ½ months aging. Aerosol formulation remains clear after 12 months aging.

### Example 10

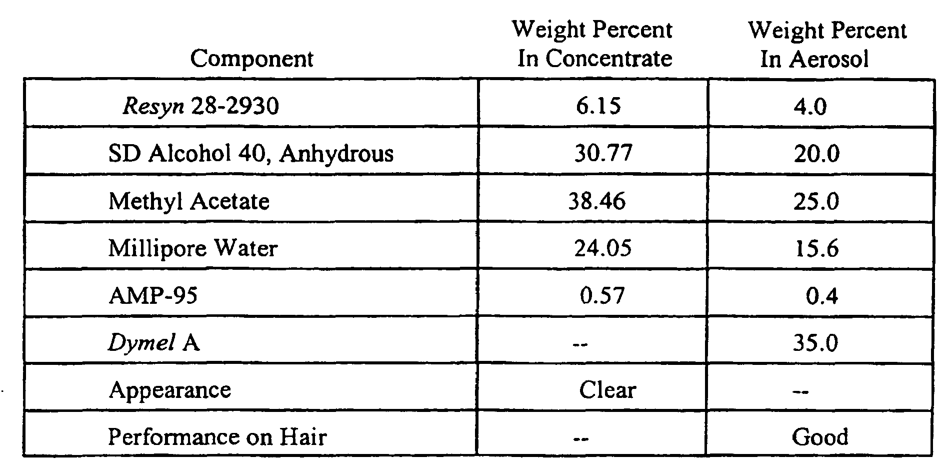

Analysis of the concentrate indicated 0.26 weight % acetic acid after 5 months aging, and 1.0 weight % acetic acid after 12 months aging. Formulation remains clear after 12 months aging.

### Example 11

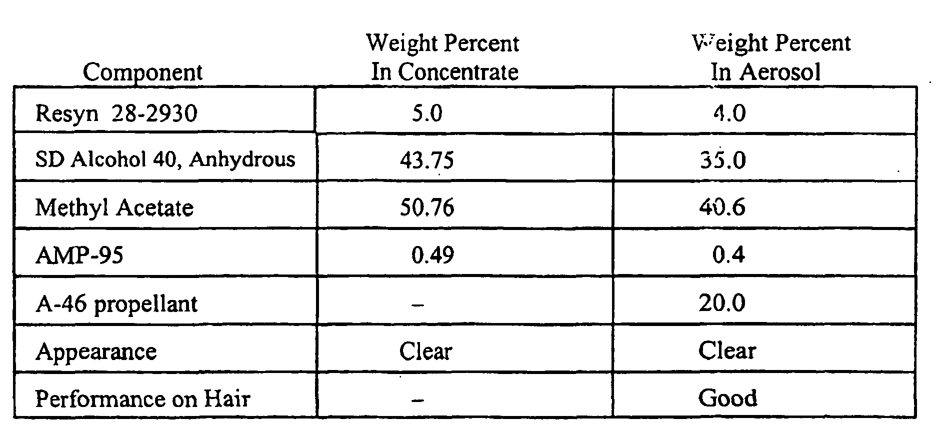

## Claims

1. A hair care composition comprising:
a. from 1 to 12 weight % fixative;
b. from 20 to 55 weight % ethanol; and
c. from 1 to 45 weight % methyl acetate and/or t-butyl acetate, wherein the volatile organic compound content of the composition is not higher than 55%.

2. The hair care composition of claim 1 comprising:
a. from 4 to 8 weight % fixative;
b. from 30 to 55 weight % ethanol; and
c. from 10 to 40 weight % methyl acetate.

3. The hair care composition of claim 1 wherein the fixative comprises:
a. a polymer or copolymer of acrylic acid and/or methacrylic acid or one of their polymerizable esters;
b. a polymer or copolymer of acrylamide, hydroxy acrylate, t-butylaminoethyl methacrylate, octyl acrylate, octylacrylamide, vinyl caprolactam, crotonic acid, dimethylaminopropylacrylamide, vinylpyrrolidone, vinyl acetate, vinyl propionate, vinyl caprolactam, and/or dimethicone;
c. an ethyl, propyl, or butyl ester of polyvinyl methyl ether and maleic anhydride copolymer;
d. a vinyl acetate/crotonates/vinyl neodecanoate copolymer;
e. an octylacrylamide/acrylates/butylaminoethyl methacrylate copolymer; or
f. a mixture thereof.

4. The hair care composition of claim 3 wherein the fixative is a vinyl acetate/crotonates/vinyl neodecanoate copolymer or octylacrylamide/acrylates/butylaminoethyl methacrylate copolymer.

5. The hair care composition of claim 1 further comprising water, wherein the fixative is a water dispersible sulfopolyester.

6. The hair care composition of claim 1 further comprising water, wherein the fixative is a diglycol/cyclohexane dimethanol/isophthalates/sodium sulfoisophthalate copolymer.

7. The hair care composition of claim 1 further comprising a propellant.

8. The hair care composition of claim 1 further comprising water and a propellant, wherein the propellant comprises dimethyl ether.

9. The hair care composition of claim 7 further comprising a propellant, wherein the propellant comprises methane, ethane, propane, isobutane, n-butane, dimethyl ether, 1,1-difluoroethane, 1,1,1,2-tetrafluoroethane, or a mixture thereof.

10. The hair care composition of claim 9 further comprising a propellant, wherein the propellant comprises propane and isobutane.

11. The hair care composition of claim 10 further comprising a propellant, wherein the propellant comprises from 5 to 50 parts by weight propane, and from 50 to 95 parts by weight isobutane.

12. The hair care composition of claim 7 further comprising a propellant, wherein the propellant comprises 1,1-difluoroethane.

13. The hair care composition of claim 1 comprising:
a. from 1 to 10 weight % fixative;
b. from 20 to 55 weight % ethanol;
c. from 1 to 45 weight % methyl acetate; and
d. from 15 to 45 weight % propellant.

14. The hair care composition of claim 13 comprising:
a. from 2 to 8 weight % fixative;
b. from 20 to 55 weight % ethanol;
c. from 10 to 40 weight % methyl acetate; and
d. from 20 to 35 weight % propellant.

15. The hair care composition of claim 1 further comprising water.

16. The hair care composition of claim 15 comprising:
a. from 1 to 12 weight % fixative;
b. from 20 to 55 weight % ethanol;
c. from 1 to 45 weight % methyl acetate; and
d. from 0.01 to 45 weight % water.

17. The hair care composition of claim 16 comprising:
a. from 4 to 8 weight % fixative;
b. from 30 to 55 weight % ethanol;
c. from 10 to 40 weight % methyl acetate; and
d. from 0.01 to 30 weight % water.

18. The hair care composition of claim 1 comprising:
a. from 1 to 10 weight % fixative;
b. from 20 to 55 weight % ethanol;
c. from 1 to 45 weight % methyl acetate;
d. from 0.01 to 45 weight % water; and
e. from 15 to 45 weight % propellant.

19. The hair care composition of claim 18 comprising:
a. from 2 to 8 weight % fixative;
b. from 20 to 55 weight % ethanol;
c. from 10 to 40 weight % methyl acetate;
d. from 0.01 to 30 weight % water; and
e. from 20 to 35 weight % propellant.

20. The hair care composition of claim 1 further comprising water, wherein the composition comprises from 5 to 10 weight % methyl acetate, and the molar ratio of water to ethanol does not exceed 10.

21. The hair care composition of claim 1 further comprising water, wherein the composition comprises from 11 to 15 weight % methyl acetate, and the molar ratio of water to ethanol does not exceed 6.

22. The hair care composition of claim 1 further comprising water, wherein the composition comprises from 16 to 20 weight % methyl acetate, and the molar ratio of water to ethanol does not exceed 3.5.

23. The hair care composition of claim 1 further comprising water, wherein the composition comprises from 21 to 25 weight % methyl acetate, and the molar ratio of water to ethanol does not exceed 2.5.

24. The hair care composition of claim 1 further comprising water, wherein the composition comprises from 26 to 30 weight % methyl acetate, and the molar ratio of water to ethanol does not exceed 2.0.

25. The hair care composition of claim 1 further comprising water, wherein the composition comprises from 31 to 40 weight % methyl acetate, and the molar ratio of water to ethanol does not exceed 1.6.

26. The hair care composition of claim 1 further comprising a neutralizer.

27. The hair care composition of claim 26 further comprising from 0.1 to 5 weight % of a neutralizer, wherein the neutralizer comprises aminomethyl propanediol; aminomethyl propanol; dimethylaminomethyl propanol; triisopropyl amine; dimethyl stearamine; dimethyl hydrogenated tallow amine; triethanolamine; tris(hydroxymethyl)aminomethane; sodium hydroxide; potassium hydroxide; ammonium hydroxide; or diethylpropylamine.

28. The hair care composition of claim 26 further comprising from 0.1 to 5 weight % of a neutralizer, wherein the neutralizer comprises 2-amino, 2-methyl, 1-propanol.

29. The hair care composition of claim 1 substantially free of any additional organic nonpropellant solvents.

30. The hair care composition of claim 1 selected from the group consisting of a spritz, a spray gel, a setting lotion, a glaze, and a mousse.

31. The hair care composition of claim 1 selected from the group consisting of a hair spray.

32. The hair care composition according to claim 1 comprising:
a. from 1 to 7 weight % fixative;
b. from 20 to 55 weight % ethanol; and
c. from 1 to 40 weight % methyl acetate,
wherein the volatile organic compound content of the composition is not higher than 55%.

33. A consumer article comprising:
a. a hand-held spray container; and
b. a sprayable composition contained within the spray container comprising:
i. from 20 to 55 weight % ethanol; and
ii. from 1 to 45 weight % methyl acetate and/or t-butyl acetate,
wherein the volatile organic compound content of the composition is not higher than 55%.

34. The consumer article of claim 33 wherein the sprayable composition comprises:
a. from 30 to 55 weight % ethanol; and
b. from 10 to 40 weight % methyl acetate.

35. The consumer article of claim 33 wherein the sprayable composition comprises:
a. from 20 to 55 weight % ethanol;
b. from 1 to 45 weight % methyl acetate; and
c. from 15 to 60 weight % propellant.

36. The consumer article of claim 35 wherein the sprayable composition comprises:
a. from 20 to 55 weight % ethanol;
b. from 10 to 40 weight % methyl acetate; and
c. from 20 to 35 weight % propellant.

37. The consumer article of claim 33 wherein the sprayable composition further comprises from 0.01 to 45 weight % water.

38. The consumer article of claim 37 wherein the sprayable composition further comprises from 0.01 to 20 weight % water.

39. The consumer article of claim 33 wherein the sprayable composition further comprises a perspiration deodorant, antiperspirant, or mixture thereof.

40. The consumer article of claim 33 wherein the sprayable composition further comprises a room or surface deodorant, disinfectant, fragrance, or mixture thereof.

41. A consumer article comprising:
a. a hand-held spray container; and
b. a sprayable composition contained within the spray container comprising:
i. from 20 to 55 weight % isopropanol; and
ii. from 1 to 45 weight % methyl acetate and/or t-butyl acetate,
wherein the volatile organic compound content of the composition is not higher than 55%.

42. The consumer article of claim 41 wherein the sprayable composition comprises:
a. from 30 to 55 weight % isopropanol; and
b. from 10 to 40 weight % methyl acetate.

43. The consumer article of claim 41 wherein the sprayable composition comprises:
a. from 20 to 55 weight % isopropanol;
b. from 1 to 45 weight % methyl acetate; and
c. from 15 to 60 weight % propellant.

44. The consumer article of claim 43 wherein the sprayable composition comprises:
a. from 20 to 55 weight % isopropanol;
b. from 10 to 40 weight % methyl acetate; and
c. from 20 to 35 weight % propellant.

45. A method of fixing hair comprising spraying the composition of claim 1 onto hair.

## Patentansprüche

1. Haarpflegezusammensetzung, umfassend:
a. von 1 bis 12 Gew.-% Haarfestiger;
b. von 20 bis 55 Gew.-% Ethanol; und
c. von 1 bis 45 Gew.-% Methylacetat und/oder t-Butylacetat,
wobei der Gehalt an flüchtigen organischen Verbindungen der Zusammensetzung nicht höher als 55 % liegt.

2. Haarpflegezusammensetzung gemäß Anspruch 1, umfassend:
a. von 4 bis 8 Gew.-% Haarfestiger;
b. von 30 bis 55 Gew.-% Ethanol; und
c. von 10 bis 40 Gew.-% Methylacetat.

3. Haarpflegezusammensetzung gemäß Anspruch 1, wobei der Haarfestiger folgendes umfasst:
a. ein Polymer oder Copolymer von Acrylsäure und/oder Methacrylsäure oder von einem ihrer polymerisierbaren Ester;
b. ein Polymer oder Copolymer von Acrylamid, Hydroxyacrylat, t-Butylaminoethylmethacrylat, Octylacrylat, Octylacrylamid, Vinylcaprolactam, Crotonsäure, Dimethylaminopropylacrylamid, Vinylpyrrolidon, Vinylacetat, Vinylpropionat, Vinylcaprolactam und/oder Dimethicon;
c. einen Ethyl-, Propyl- oder Butylester von Polyvinylmethylether und Maleinsäureanhydrid-Copolymer;
d. ein Vinylacetat/Crotonate/Vinylneodecanoat-Copolymer;
e. ein Octylacrylamid/Acrylate/Butylaminoethylmethacrylat-Copolymer; oder
f. eine Mischung davon.

4. Haarpflegezusammensetzung gemäß Anspruch 3, wobei der Haarfestiger ein Vinylacetat/Crotonate/Vinylneodecanoat-Copolymer oder Octylacrylamid/Acrylate/Butylaminoethylmethacrylat-Copolymer ist.

5. Haarpflegezusammensetzung gemäß Anspruch 1, ferner Wasser umfassend, wobei der Haarfestiger ein in Wasser dispergierbarer Sulfopolyester ist.

6. Haarpflegezusammensetzung gemäß Anspruch 1, ferner Wasser umfassend, wobei der Haarfestiger ein Diglycol/Cyclohexandimethanol/Isophthalate/Natriumsulfoisophthalat-Copolymer ist.

7. Haarpflegezusammensetzung gemäß Anspruch 1, ferner ein Treibmittel umfassend.

8. Haarpflegezusammensetzung gemäß Anspruch 1, ferner Wasser und ein Treibmittel umfassend, wobei das Treibmittel Dimethylether umfasst.

9. Haarpflegezusammensetzung gemäß Anspruch 7, ferner ein Treibmittel umfassend, wobei das Treibmittel Methan, Ethan, Propan, Isobutan, n-Butan, Dimethylether, 1,1-Difluorethan, 1,1,1,2-Tetrafluorethan oder eine Mischung davon umfasst.

10. Haarpflegezusammensetzung gemäß Anspruch 9, ferner ein Treibmittel umfassend, wobei das Treibmittel Propan und Isobutan umfasst.

11. Haarpflegezusammensetzung gemäß Anspruch 10, ferner ein Treibmittel umfassend, wobei das Treibmittel 5 bis 50 Gewichtsteile Propan und 50 bis 95 Gewichtsteile Isobutan umfasst.

12. Haarpflegezusammensetzung gemäß Anspruch 7, ferner ein Treibmittel umfassend, wobei das Treibmittel 1,1-Difluorethan umfasst.

13. Haarpflegezusammensetzung gemäß Anspruch 1, umfassend:
a. von 1 bis 10 Gew.-% Haarfestiger;
b. von 20 bis 55 Gew.-% Ethanol;
c. von 1 bis 45 Gew.-% Methylacetat; und
d. von 15 bis 45 Gew.-% Treibmittel.

14. Haarpflegezusammensetzung gemäß Anspruch 13, umfassend:
a. von 2 bis 8 Gew.% Haarfestiger;
b. von 20 bis 55 Gew.% Ethanol;
c. von 10 bis 40 Gew.% Methylacetat; und
d. von 20 bis 35 Gew.% Treibmittel.

15. Haarpflegezusammensetzung gemäß Anspruch 1, ferner Wasser umfassend.

16. Haarpflegezusammensetzung gemäß Anspruch 15, umfassend:
a. von 1 bis 12 Gew.-% Haarfestiger;
b. von 20 bis 55 Gew.-% Ethanol;
c. von 1 bis 45 Gew.-% Methylacetat; und
d. von 0,01 bis 45 Gew.-% Wasser.

17. Haarpflegezusammensetzung gemäß Anspruch 16, umfassend:
a. von 4 bis 8 Gew.-% Haarfestiger;
b. von 30 bis 55 Gew.-% Ethanol;
c. von 10 bis 40 Gew.-% Methylacetat; und
d. von 0,01 bis 30 Gew.-% Wasser.

18. Haarpflegezusammensetzung gemäß Anspruch 1, umfassend:
a. von 1 bis 10 Gew.-% Haarfestiger;
b. von 20 bis 55 Gew.-% Ethanol;
c. von 1 bis 45 Gew.-% Methylacetat;
d. von 0,01 bis 45 Gew.-% Wasser; und
e. von 15 bis 45 Gew.-% Treibmittel.

19. Haarpflegezusammensetzung gemäß Anspruch 18, umfassend:
a. von 2 bis 8 Gew.-% Haarfestiger;
b. von 20 bis 55 Gew.-% Ethanol;
c. von 10 bis 40 Gew.-% Methylacetat;
d. von 0,01 bis 30 Gew.-% Wasser; und
e. von 20 bis 35 Gew.-% Treibmittel.

20. Haarpflegezusammensetzung gemäß Anspruch 1, ferner Wasser umfassend, wobei die Zusammensetzung 5 bis 10 Gew.-% Methylacetat umfasst und das Molverhältnis von Wasser zu Ethanol 10 nicht übersteigt.

21. Haarpflegezusammensetzung gemäß Anspruch 1, ferner Wasser umfassend, wobei die Zusammensetzung 11 bis 15 Gew.% Methylacetat umfasst und das Molverhältnis von Wasser zu Ethanol 6 nicht übersteigt.

22. Haarpflegezusammensetzung gemäß Anspruch 1, ferner Wasser umfassend, wobei die Zusammensetzung 16 bis 20 Gew.-% Methylacetat umfasst und das Molverhältnis von Wasser zu Ethanol 3,5 nicht übersteigt.

23. Haarpflegezusammensetzung gemäß Anspruch 1, ferner Wasser umfassend, wobei die Zusammensetzung 21 bis 25 Gew.-% Methylacetat umfasst und das Molverhältnis von Wasser zu Ethanol 2,5 nicht übersteigt.

24. Haarpflegezusammensetzung gemäß Anspruch 1, ferner Wasser umfassend, wobei die Zusammensetzung 26 bis 30 Gew.-% Methylacetat umfasst und das Molverhältnis von Wasser zu Ethanol 2,0 nicht übersteigt.

25. Haarpflegezusammensetzung gemäß Anspruch 1, ferner Wasser umfassend, wobei die Zusammensetzung 31 bis 40 Gew.-% Methylacetat umfasst und das Molverhältnis von Wasser zu Ethanol 1,6 nicht übersteigt.

26. Haarpflegezusammensetzung gemäß Anspruch 1, ferner einen Neutralisator umfassend.

27. Haarpflegezusammensetzung gemäß Anspruch 26, ferner 0,1 bis 5 Gew.-% eines Neutralisators umfassend, wobei der Neutralisator Aminomethylpropandiol; Aminomethylpropanol; Dimethylaminomethylpropanol; Triisopropylamin; Dimethylstearamin; hydriertes Dimethyltalgamin; Triethanolamin; Tris(hydroxymethyl)aminomethan; Natriumhydroxid; Kaliumhydroxid; Ammmoniumhydroxid oder Diethylpropylamin umfasst.

28. Haarpflegezusammensetzung gemäß Anspruch 26, ferner 0,1 bis 5 Gew.-% eines Neutralisators umfassend, wobei der Neutralisator 2-Amino-2-methyl-1-propanol umfasst.

29. Haarpflegezusammensetzung gemäß Anspruch 1, die im Wesentlichen frei von irgendwelchen zusätzlichen organischen Nicht-Treibmittel-Lösungsmitteln ist.

30. Haarpflegezusammensetzung gemäß Anspruch 1, gewählt aus der Gruppe, bestehend aus einer Spritzlösung, einem Sprühgel, einer Festigungslotion, einem Glanzmittel und einem Aufbauschaum.

31. Haarpflegezusammensetzung gemäß Anspruch 1, gewählt aus der Gruppe, bestehend aus einem Haarspray.

32. Haarpflegezusammensetzung gemäß Anspruch 1, umfassend:
a. von 1 bis 7 Gew.-% Haarfestiger;
b. von 20 bis 55 Gew.-% Ethanol; und
c. von 1 bis 40 Gew.-% Methylacetat,
wobei der Gehalt an flüchtigen organischen Verbindungen der Zusammensetzung nicht höher als 55 % liegt.

33. Verbraucherartikel, umfassend:
a. einen Handsprühbehälter; und
b. eine sprühfähige Zusammensetzung, welche innerhalb des Sprühbehälters enthalten ist, umfassend:
i. von 20 bis 55 Gew.-% Ethanol; und
ii. von 1 bis 45 Gew.-% Methylacetat und/oder t-Butylacetat,
wobei der Gehalt an flüchtigen organischen Verbindungen der Zusammensetzung nicht höher als 55 % liegt.

34. Verbraucherartikel gemäß Anspruch 33, wobei die sprühfähige Zusammensetzung folgendes umfasst:
a. von 30 bis 55 Gew.-% Ethanol; und
b. von 10 bis 40 Gew.-% Methylacetat.

35. Verbraucherartikel gemäß Anspruch 33, wobei die sprühfähige Zusammensetzung folgendes umfasst:
a. von 20 bis 55 Gew.-% Ethanol;
b. von 1 bis 45 Gew.-% Methylacetat; und
c. von 15 bis 60 Gew.-% Treibmittel.

36. Verbraucherartikel gemäß Anspruch 35, wobei die sprühfähige Zusammensetzung folgendes umfasst:
a. von 20 bis 55 Gew.-% Ethanol;
b. von 10 bis 40 Gew.-% Methylacetat; und
c. von 20 bis 35 Gew.-% Treibmittel.

37. Verbraucherartikel gemäß Anspruch 33, wobei die sprühfähige Zusammensetzung ferner 0,01 bis 45 Gew.-% Wasser umfasst.

38. Verbraucherartikel gemäß Anspruch 37, wobei die sprühfähige Zusammensetzung ferner 0,01 bis 20 Gew.-% Wasser umfasst.

39. Verbraucherartikel gemäß Anspruch 33, wobei die sprühfähige Zusammensetzung ferner ein Schweißdeodorant, Antitranspirant oder eine Mischung davon umfasst.

40. Verbraucherartikel gemäß Anspruch 33, wobei die sprühfähige Zusammensetzung ferner ein Raum- oder Oberflächendeodorant, Desinfektionsmittel, Duftstoff oder eine Mischung davon umfasst.

41. Verbraucherartikel, umfassend:
a. einen Handsprühbehälter; und
b. eine sprühfähige Zusammensetzung, welche innerhalb des Sprühbehälters enthalten ist, umfassend:
i. von 20 bis 55 Gew.-% Isopropanol; und
ii. von 1 bis 45 Gew.-% Methylacetat und/oder t-Butylacetat,
wobei der Gehalt an flüchtigen organischen Verbindungen der Zusammensetzung nicht höher als 55 % liegt.

42. Verbraucherartikel gemäß Anspruch 41, wobei die sprühfähige Zusammensetzung folgendes umfasst:
a. von 30 bis 55 Gew.-% Isopropanol; und
b. von 10 bis 40 Gew.-% Methylacetat.

43. Verbraucherartikel gemäß Anspruch 41, wobei die sprühfähige Zusammensetzung folgendes umfasst:
a. von 20 bis 55 Gew.-% Isopropanol;
b. von 1 bis 45 Gew.-% Methylacetat; und
c. von 15 bis 60 Gew.-% Treibmittel.

44. Verbraucherartikel gemäß Anspruch 43, wobei die sprühfähige Zusammensetzung folgendes umfasst:
a. von 20 bis 55 Gew.-% Isopropanol;
b. von 10 bis 40 Gew.-% Methylacetat; und
c. von 20 bis 35 Gew.-% Treibmittel.

45. Verfahren zum Fixieren von Haar, umfassend das Sprühen der Zusammensetzung von Anspruch 1 auf das Haar.

## Revendications

1. Composition de soin capillaire comprenant :
a. de 1 à 12 % en poids de fixateur ;
b. de 20 à 55 % en poids d'éthanol ; et
c. de 1 à 45 % en poids d'acétate de méthyle et/ou d'acétate de t-butyle,
dans laquelle la teneur en composés organiques volatils de la composition n'est pas supérieure à 55 %.

2. Composition de soin capillaire selon la revendication 1, comprenant :
a. de 4 à 8 % en poids de fixateur ;
b. de 30 à 55 % en poids d'éthanol ; et
c. de 10 à 40 % en poids d'acétate de méthyle.

3. Composition de soin capillaire selon la revendication 1, dans laquelle le fixateur comprend :
a. un polymère ou un copolymère d'acide acrylique et/ou d'acide méthacrylique ou d'un de leurs esters polymérisables ;
b. un polymère ou un copolymère d'acrylamide, d'hydroxyacrylate, de méthacrylate de t-butylaminoéthyle, d'acrylate d'octyle, d'octylacrylamide, de vinylcaprolactame, d'acide crotonique, de diméthylaminopropylacrylamide, de vinylpyrrolidone, d'acétate de vinyle, de propionate de vinyle, de vinylcaprolactame et/ou de diméthicone ;
c. un copolymère d'ester éthylique, propylique ou butylique de polyvinylméthyléther et d'anhydride maléique ;
d. un copolymère d'acétate de vinyle/crotonates/ néodécanoate de vinyle ;
e. un copolymère d'octylacrylamide/acrylates/méthacrylate butylaminoéthyle ; ou
f. un mélange de ceux-ci.

4. Composition de soin capillaire selon la revendication 3, dans laquelle le fixateur est un copolymère d'acétate de vinyle/crotonates/néodécanoate de vinyle ou un copolymère d'octylacrylamide/acrylates/méthacrylate de butylaminoéthyle.

5. Composition de soin capillaire selon la revendication 1, comprenant en outre de l'eau, dans laquelle le fixateur est un sulfopolyester dispersible dans l'eau.

6. Composition de soin capillaire selon la revendication 1, comprenant en outre de l'eau, dans laquelle le fixateur est un copolymère de diglycol/cyclohexane diméthanol/isophtalates/sulfo-isophtalate de sodium.

7. Composition de soin capillaire selon la revendication 1, comprenant en outre un propulseur.

8. Composition de soin capillaire selon la revendication 1, comprenant en outre de l'eau et un propulseur, dans laquelle le propulseur comprend de l'éther diméthylique.

9. Composition de soin capillaire selon la revendication 7, comprenant en outre un propulseur, dans laquelle le propulseur comprend du méthane, de l'éthane, du propane, de l'isobutane, du n-butane, de l'éther diméthylique, du 1,1-difluoroéthane, du 1,1,1,2-tétrafluoroéthane ou un mélange de ceux-ci.

10. Composition de soin capillaire selon la revendication 9, comprenant en outre un propulseur, dans laquelle le propulseur comprend du propane et de l'isobutane.

11. Composition de soin capillaire selon la revendication 10, comprenant en outre un propulseur, dans laquelle le propulseur comprend de 5 à 50 parties en poids de propane et de 50 à 95 parties en poids d'isobutane.

12. Composition de soin capillaire selon la revendication 7, comprenant en outre un propulseur, dans laquelle le propulseur comprend du 1,1-difluoroéthane.

13. Composition de soin capillaire selon la revendication 1, comprenant :
a. de 1 à 10 % en poids de fixateur ;
b. de 20 à 55 % en poids d'éthanol ;
c. de 1 à 45 % en poids d'acétate de méthyle ; et
d. de 15 à 45 % en poids de propulseur.

14. Composition de soin capillaire selon la revendication 13, comprenant :
a. de 2 à 8 % en poids de fixateur ;
b. de 20 à 55 % en poids d'éthanol ;
c. de 10 à 40 % en poids d'acétate de méthyle ; et
d. de 20 à 35 % en poids de propulseur.

15. Composition de soin capillaire selon la revendication 1, comprenant en outre de l'eau.

16. Composition de soin capillaire selon la revendication 15, comprenant :
a. de 1 à 12 % en poids de fixateur ;
b. de 20 à 55 % en poids d'éthanol ;
c. de 1 à 45 % en poids d'acétate de méthyle ; et
d. de 0,01 à 45 % en poids d'eau.

17. Composition de soin capillaire selon la revendication 16, comprenant :
a. de 4 à 8 % en poids de fixateur ;
b. de 30 à 55 % en poids d'éthanol ;
c. de 10 à 40 % en poids d'acétate de méthyle ; et
d. de 0,01 à 30 % en poids d'eau.

18. Composition de soin capillaire selon la revendication 1, comprenant :
a. de 1 à 10 % en poids de fixateur ;
b. de 20 à 55 % en poids d'éthanol ;
c. de 1 à 45 % en poids d'acétate de méthyle ;
d. de 0,01 à 45 % en poids d'eau ; et
e. de 15 à 45 % en poids de propulseur.

19. Composition de soin capillaire selon la revendication 18, comprenant :
a. de 2 à 8 % en poids de fixateur ;
b. de 20 à 55 % en poids d'éthanol ;
c. de 10 à 40 % en poids d'acétate de méthyle;
d. de 0,01 à 30 % en poids d'eau ; et
e. de 20 à 35 % en poids de propulseur.

20. Composition de soin capillaire selon la revendication 1, comprenant en outre de l'eau, dans laquelle la composition comprend de 5 à 10 % en poids d'acétate de méthyle, et le rapport molaire de l'eau à l'éthanol ne dépasse pas 10.

21. Composition de soin capillaire selon la revendication 1, comprenant en outre de l'eau, dans laquelle la composition comprend de 11 à 15 % en poids d'acétate de méthyle, et le rapport molaire de l'eau à l'éthanol ne dépasse pas 6.

22. Composition de soin capillaire selon la revendication 1, comprenant en outre de l'eau, dans laquelle la composition comprend de 16 à 20 % en poids d'acétate de méthyle, et le rapport molaire de l'eau à l'éthanol ne dépasse pas 3,5.

23. Composition de soin capillaire selon la revendication 1, comprenant en outre de l'eau, dans laquelle la composition comprend de 21 à 25 % en poids d'acétate de méthyle, et le rapport molaire de l'eau à l'éthanol ne dépasse pas 2,5.

24. Composition de soin capillaire selon la revendication 1, comprenant en outre de l'eau, dans laquelle la composition comprend de 26 à 30 % en poids d'acétate de méthyle, et le rapport molaire de l'eau à l'éthanol ne dépasse pas 2,0.

25. Composition de soin capillaire selon la revendication 1, comprenant en outre de l'eau, dans laquelle la composition comprend de 31 à 40 % en poids d'acétate de méthyle, et le rapport molaire de l'eau à l'éthanol ne dépasse pas 1,6.

26. Composition de soin capillaire selon la revendication 1, comprenant en outre un agent de neutralisation.

27. Composition de soin capillaire selon la revendication 26, comprenant en outre de 0,1 à 5 % en poids d'un agent de neutralisation, dans laquelle l'agent de neutralisation comprend de l'aminométhylpropanediol ; de l'aminométhylpropanol ; du diméthylaminométhylpropanol ; de la triisopropylamine ; de la diméthylstéarylamine ; de la diméthylamine de suif hydrogénée ; de la triéthanolamine ; du tris(hydroxyméthyl)aminométhane ; de l'hydroxyde de sodium ; de l'hydroxyde de potassium ; de l'hydroxyde d'ammonium ; ou de la diéthylpropylamine.

28. Composition de soin capillaire selon la revendication 26, comprenant en outre de 0,1 à 5 % en poids d'un agent de neutralisation, dans laquelle l'agent de neutralisation comprend du 2-amino-2-méthyl-1-propanol.

29. Composition de soin capillaire selon la revendication 1, essentiellement dépourvue de tout solvant organique non propulseur supplémentaire.

30. Composition de soin capillaire selon la revendication 1, choisie dans le groupe constitué par un spray, un gel de pulvérisation, une lotion fixante, une laque et une mousse.

31. Composition de soin capillaire selon la revendication 1, choisie dans le groupe constitué par une pulvérisation capillaire.

32. Composition de soin capillaire selon la revendication 1, comprenant :
a. de 1 à 7 % en poids de fixateur ;
b. de 20 à 55 % en poids d'éthanol ; et
c. de 1 à 40 % en poids d'acétate de méthyle,
dans laquelle la teneur en composés organiques volatils de la composition n'est pas supérieure à 55 %.

33. Article de consommation comprenant :
a. un récipient de pulvérisation à main ; et
b. une composition pulvérisable contenue à l'intérieur du récipient de pulvérisation comprenant :
i. de 20 à 55 % en poids d'éthanol ; et
ii. de 1 à 45 % en poids d'acétate de méthyle et/ou d'acétate de t-butyle,
dans lequel la teneur en composés organiques volatils de la composition n'est pas supérieure à 55 %.

34. Article de consommation selon la revendication 33, dans lequel la composition pulvérisable comprend :
a. de 30 à 55 % en poids d'éthanol ; et
b. de 10 à 40 % en poids d'acétate de méthyle.

35. Article de consommation selon la revendication 33, dans lequel la composition pulvérisable comprend :
a. de 20 à 55 % en poids d'éthanol ;
b. de 1 à 45 % en poids d'acétate de méthyle ; et
c. de 15 à 60 % en poids de propulseur.

36. Article de consommation selon la revendication 35, dans lequel la composition pulvérisable comprend :
a. de 20 à 55 % en poids d'éthanol ;
b. de 10 à 40 % en poids d'acétate de méthyle ; et
c. dé 20 à 35 % en poids de propulseur.

37. Article de consommation selon la revendication 33, dans lequel la composition pulvérisable comprend en outre de 0,01 à 45 % d'eau.

38. Article de consommation selon la revendication 37, dans lequel la composition pulvérisable comprend en outre de 0,01 à 20 % d'eau.

39. Article de consommation selon la revendication 33, dans lequel la composition pulvérisable comprend en outre un déodorant contre la transpiration, un antitranspirant ou un mélange de ceux-ci.

40. Article de consommation selon la revendication 33, dans lequel la composition pulvérisable comprend en outre un déodorant d'ambiance ou de surface, un désinfectant, un parfum ou un mélange de ceux-ci.

41. Article de consommation comprenant :
a. un récipient de pulvérisation à main ; et
b. une composition pulvérisable contenue à l'intérieur du récipient de pulvérisation comprenant :
i. de 20 à 55 % en poids d'isopropanol ; et
ii. de 1 à 45 % en poids d'acétate de méthyle et/ou d'acétate de t-butyle,
dans lequel la teneur en composés organiques volatils de la composition n'est pas supérieure à 55 %.

42. Article de consommation selon la revendication 41, dans lequel la composition pulvérisable comprend :
a. de 30 à 55 % en poids d'isopropanol ; et
b. de 10 à 40 % en poids d'acétate de méthyle.

43. Article de consommation selon la revendication 41, dans lequel la composition pulvérisable comprend :
a. de 20 à 55 % en poids d'isopropanol ;
b. de 1 à 45 % en poids d'acétate de méthyle ; et
c. de 15 à 60 % en poids de propulseur.

44. Article de consommation selon la revendication 43, dans lequel la composition pulvérisable comprend :
a. de 20 à 55 % en poids d'isopropanol ;
b. de 10 à 40 % en poids d'acétate de méthyle ; et
c. de 20 à 35 % en poids de propulseur.

45. Procédé de fixation des cheveux comprenant la pulvérisation de la composition selon la revendication 1 sur les cheveux.
